# EUROPEAN PATENT APPLICATION

(11) **EP 4 603 512 A1**
(43) Date of publication of application: **20.08.2025**
(21) Application number: 22961736.0
(22) Date of filing: 13.10.2022
(51) Int. Cl.: C07K 16/46, C12N 15/13, C12N 15/85, A61K 39/395, A61P 35/00

(54) **B7H3/PDL1 BISPECIFIC ANTIBODY, AND PHARMACEUTICAL COMPOSITION COMPRISING SAME AND USE THEREOF**

(71) Applicant: Dartsbio Pharmaceuticals Ltd., Zhongshan, Guangdong 528400 (CN); Shanghai Mabstone Biotechnology Ltd., Shanghai 200120 (CN)
(72) Inventor: CHEN, Yili, Zhongshan City, Guangdong 528400 (CN); WANG, Chunhe, Zhongshan City, Guangdong 528400 (CN); LI, Huanhuan, Zhongshan City, Guangdong 528400 (CN); LIU, Guojian, Zhongshan City, Guangdong 528400 (CN); TAN, Jie, Zhongshan City, Guangdong 528400 (CN); TANG, Lei, Zhongshan City, Guangdong 528400 (CN)
(74) Representative: Clark, Claudia
(86) International application number: PCT/CN2022/125089
(87) International publication number: WO 2024/077547

(57) **Abstract**

The present invention relates to a B7H3/PDL1 bispecific antibody, and a pharmaceutical composition comprising same and a use thereof. The bispecific antibody can bind to B7H3 and PDL1 in a targeted manner, and comprises: a monoclonal antibody unit, targeting PDL1 and comprising two heavy chains and two light chains; and a nano-antibody unit, targeting B7H3 and comprising two identical nano-antibodies, wherein the C-terminuses of the two nano-antibodies are respectively linked to the C-terminuses of the Fc fragments of the two heavy chains of the monoclonal antibody unit by means of a linker peptide. The bispecific antibody of the present invention can bind to both B7H3 and PDL1, can relieve inhibition of T cells by PDL1 while targeting tumor cells, and shows anti-tumor activity superior to that of a drug combination using a monoclonal antibody.

## Description

### Technical field

The present invention belongs to the field of biopharmaceuticals, and specifically relates to a B7H3/PDL1 bispecific antibody and a pharmaceutical composition comprising the same and use thereof.

### Background

As a co-stimulatory signal for T lymphocyte activation, the B7-CD28 family plays a vital role in the immune response in which T lymphocytes participate. Studies have shown that different types of B7 molecules have positive or negative regulation on immune cell responses. B7H3 (also known as CD276) is a member of the B7 family and is mainly expressed on the surface of tumor cells. Chapoval Al et al. first discovered that it has a co-stimulatory effect on CD4⁺ and CD8⁺ T cells. B7H3 signaling induces cellular immunity and selectively enhances the production of interferon-γ (IFN-γ) via T cell receptor signaling. However, as research on B7H3 is currently the most extensive, the inhibitory function of B7H3 has been gradually discovered. For example, it can inhibit the proliferation of CD4⁺T and CD8⁺T cells. In addition, studies have shown that abnormal expression of B7H3 is associated with the occurrence, development and metastasis of various cancers, and there is a lot of evidence that its high expression is associated with poor prognosis in various malignancies.

B7H3 is highly expressed in all cancer types tested, with limited heterogeneity, and is rarely expressed in normal tissues. This suggests that B7H3 can be considered a tumor antigen (TA), which provides a possibility for targeted therapy of tumor cells with high B7H3 expression. At present, B7H3 targeted therapy strategies mainly include blocking monoclonal antibodies, radioimmunotherapy, antibody-drug conjugates (ADCs), monoclonal antibodies and bispecific antibodies (BsAbs) that mediate cytotoxicity, and the like. Enoblituzumab (MGA271), a humanized monoclonal antibody targeting B7H3 developed by MacroGenics, acts on a variety of malignancies such as melanoma, osteosarcoma, and Ewing's sarcoma based on antibody-dependent cell-mediated cytotoxicity (ADCC). Enoblituzumab has been shown to have strong anti-tumor activity in a variety of xenograft tumor models and has no obvious toxicity in the safety evaluation of primates. Enoblituzumab is currently in Phase II clinical research and shows good therapeutic potential.

Inhibitors targeting the immune checkpoint PD1/PDL1 are undoubtedly the focus of tumor immunotherapy. PDL1 is expressed on the surface of tumor cells, and cytotoxic PDL1 inhibitory antibodies theoretically have better anti-tumor effects. However, it is reported that only Avelumab among the PDL1 monoclonal antibodies on the market can mediate ADCC effects against tumors and show safety comparable to other PDL1 antibodies. An important reason is that the effect of ADCC mainly depends on the abundance of antigen expression, while PDL1 cannot be regarded as a typical tumor antigen, and it has great heterogeneity in tumor cells.

PD1/PDL1 pathway-blocking antibodies are often used in combination with cytotoxic antibodies to enhance the effect of immunotherapy. Enoblituzumab and Pembrolizumab (a monoclonal antibody targeting PD1) are used in combination. On the one hand, they can block the immunosuppression of the PD1/PDL1 pathway, and on the other hand, they can exert tumor killing effects through Enoblituzumab. This combination therapy has entered clinical phase II (NCT04129320). Data show that the incidence of adverse reactions in the combination therapy is similar to that of monotherapy, and the objective response rate (ORR) of the combination therapy is better than that of PD1 monoclonal antibody therapy. This proves the feasibility of blocking both the B7H3 pathway and the PD1/PDL1 pathway at the same time.

Bispecific antibodies (BsAb) are genetically engineered antibodies that can simultaneously target two different antigenic epitopes. Bispecific antibodies targeting B7H3 have been developed, most of which rely on T cell redirection strategies, such as CD3/B7H3 bispecific antibodies. However, in theory, cytotoxicity-based bispecific antibodies may better act on B7H3-high-expressing tumor cells because B7H3 is expressed as a tumor antigen with characteristics of high abundance and low heterogeneity. At present, only 5 bispecific antibody drugs have been approved for marketing, and the main reason limiting their application is stability and activity issues. Therefore, efforts are still needed to be made in the industry to obtain bispecific antibodies with good stability and excellent biological activity.

### Summary of the invention

One technical purpose of the present invention is to provide a method for preparing a B7H3/PDL1 bispecific antibody and use thereof.

In one aspect, the present invention provides a B7H3/PDL1 bispecific antibody targeting B7H3 and PDL1, and the bispecific antibody comprises:
a monoclonal antibody unit, which targets PDL1 and comprises 2 heavy chains and 2 light chains;
a nanobody unit, which targets B7H3 and comprises 2 identical nanobodies (VHH),
wherein the C-termini of the two nanobodies are linked to the C-termini of the Fc fragments of the two heavy chains of the monoclonal antibody unit via a linker peptide, respectively.

In the present invention, the linker peptide refers to a polypeptide comprising a glycine and a serine and having certain elasticity and protease resistance.

In one embodiment, the amino acid sequence of the linker peptide is set forth in SEQ ID No.: 11.

In one embodiment, the light chain variable region of the monoclonal antibody unit comprises CDR1 with an amino acid sequence of SEQ ID NO.: 1, CDR2 with an amino acid sequence of SEQ ID NO.: 2, and CDR3 with an amino acid sequence of SEQ ID NO.: 3, the heavy chain variable region of the monoclonal antibody unit comprises CDR1 with an amino acid sequence of SEQ ID NO.: 5, CDR2 with an amino acid sequence of SEQ ID NO.: 6, and CDR3 with an amino acid sequence of SEQ ID NO.: 7, and the nanobody comprises CDR1 with an amino acid sequence of SEQ ID NO.: 12, CDR2 with an amino acid sequence of SEQ ID NO.: 13, and CDR3 with an amino acid sequence of SEQ ID NO.: 14.

In one embodiment, the light chain variable region of the monoclonal antibody unit comprises an amino acid sequence set forth in SEQ ID NO.: 4, and the heavy chain variable region of the monoclonal antibody unit comprises an amino acid sequence set forth in SEQ ID NO.: 8.

In one embodiment, the light chain of the monoclonal antibody unit comprises an amino acid sequence set forth in SEQ ID NO.: 9, and the heavy chain of the monoclonal antibody unit comprises an amino acid sequence set forth in SEQ ID NO.: 10.

In one embodiment, the nanobody comprises an amino acid sequence set forth in SEQ ID NO.: 15.

In one embodiment, the amino acid sequence of the light chain variable region of the monoclonal antibody unit is set forth in SEQ ID NO.: 4, and the amino acid sequence of the heavy chain variable region of the monoclonal antibody unit is set forth in SEQ ID NO.: 8.

In one embodiment, the full-length amino acid sequence of the light chain of the monoclonal antibody unit is set forth in SEQ ID NO.: 9, and the full-length amino acid sequence of the heavy chain of the monoclonal antibody unit is set forth in SEQ ID NO.: 10.

In one embodiment, the amino acid sequence of the nanobody is set forth in SEQ ID NO.: 15.

In one embodiment, the amino acid sequence of the heavy chain of the bispecific antibody is set forth in SEQ ID NO.: 16, and the amino acid sequence of the light chain of the bispecific antibody is set forth in SEQ ID NO.: 17.

In another aspect, the present invention also provides a polynucleotide encoding the B7H3/PDL1 bispecific antibody.

In another aspect, the present invention also provides an expression vector comprising a polynucleotide encoding the B7H3/PDL1 bispecific antibody.

In yet another aspect, the present invention provides a pharmaceutical composition comprising a therapeutically effective amount of the above-mentioned B7H3/PDL1 bispecific antibody and a pharmaceutically acceptable carrier.

In yet another aspect, the present invention provides a use of the B7H3/PDL1 bispecific antibody in the preparation of a drug for the prevention, diagnosis, treatment or adjuvant treatment of tumors.

In a specific embodiment, the drug inhibits tumors by binding to B7H3, blocking the B7H3 signaling pathway, and thereby mediating the ADCC effect.

In a specific embodiment, the drug inhibits tumors by binding to PD-L1, blocking the binding of PD-1 to PDL-1, activating T lymphocytes, and increasing the expression of IL-2, IFN-y in T lymphocytes.

In a specific embodiment, the drug inhibits tumors by binding to B7H3, blocking the B7H3 signaling pathway, and by binding to PD-L1, blocking the binding of PD-1 to PDL-1, activating T lymphocytes, and increasing the expression of IL-2, IFN-y in T lymphocytes.

In a specific embodiment, the tumor can be selected from one or more of lung cancer, gastric cancer, liver cancer, colorectal cancer, melanoma, kidney tumor, ovarian cancer, prostate cancer, bladder cancer, breast cancer, esophageal cancer, colorectal cancer, nasopharyngeal cancer, brain tumor, cervical cancer, blood cancer, bone cancer, lymphoma, pancreatic cancer and Ewing's sarcoma. In particular, the tumor is breast cancer, ovarian cancer or melanoma.

### Beneficial effects

The bispecific antibody constructed in the present application can bind to B7H3 and PDL1 simultaneously, and can relieve the inhibition of PDL1 on T cells while targeting tumor cells, and also show better anti-tumor activity as compared with monoclonal antibody combination therapy.

In addition, compared with related monoclonal antibody combination therapy, the bispecific antibody in the present application has the advantages of good compliance and controllable quality.

Further, the stability characterization of the bispecific antibody in the present application is mainly reflected in the study of monomer purity and thermostability. After a single affinity purification, the monomer content of the bispecific antibody can reach 95%, which is even better than the purity after multiple secondary purifications in the industry. The results of the structural and activity analysis of the antibody after heat treatment prove that the antibody can still maintain a good molecular conformation and complete biological activity under harsh conditions, which is beneficial for industrial production, packaging and storage of antibodies. In general, the present application has constructed a B7H3/PDL1 bispecific antibody in the format of IgG-VHH₂, which shows good molecular stability and has significantly better in vitro activity (binding molecular level and cell level) than Avelumab and MGA271. In vivo data show that in B7H3⁺ A375 tumor cells, the anti-tumor activity of the bispecific antibody is superior to that of the B7H3 monoclonal antibody combination group. Therefore, the bispecific antibody of the present application has broad application prospects due to its excellent developability and activity.

In summary, the method used in the present invention is a new frontier method for treating tumors at present, and tumor immunotherapy is expected to become an innovation in the field of tumor treatment after surgery, chemotherapy, radiotherapy, and targeted therapy. The B7H3/PDL1 bispecific antibody in the present invention is expected to be developed into a new anti-tumor drug.

### Brief description of the drawings

Figure 1 shows a graph showing the screening of the affinity of the anti-B7H3 VHH humanized antibody prepared in the present application and B7H3.
Figure 2 shows a structural schematic diagram of the B7H3/PDL1 bispecific antibody prepared in the present application.
Figure 3 shows a SDS-polyacrylamide gel electrophoresis diagram of the B7H3/PDL1 bispecific antibody of the present application.
Figure 4 shows the SEC-HPLC purity determination of the B7H3/PDL1 bispecific antibody of the present application.
Figure 5 shows the Tm value determination (DSF) of the B7H3/PDL1 bispecific antibody of the present application.
Figure 6 shows the binding ELISA of the B7H3/PDL1 bispecific antibody of the present application before and after heat treatment at 60°C, wherein a represents the binding ELISA with PDL1, and b represents the binding ELISA with B7H3.
Figure 7 shows the binding ELISA of the B7H3/PDL1 bispecific antibody of the present application, wherein a represents the binding ELISA with PDL1, and b represents the binding ELISA with B7H3.
Figure 8 shows a BLI affinity test diagram of the B7H3/PDL1 bispecific antibody of the present application with B7H3-his or PDL1-his at 5 different concentrations, wherein a represents the affinity curve with PDL1, and b represents the affinity curve with B7H3.
Figure 9 shows a PDL1/CHO-PD1 blocking curve of the B7H3/PDL1 bispecific antibody of the present application.
Figure 10 shows that the B7H3/PDL1 bispecific antibody of the present application induces T cells to secrete IFN-y.
Figure 11 shows that the B7H3/PDL1 bispecific antibody of the present application promotes T cell proliferation.
Figure 12 shows the ADCC effect of the B7H3/PDL1 bispecific antibody of the present application on cancer cells, a represents the cytotoxic effect on human breast cancer cells MDA-MB-231, and b represents the cytotoxic effect on human ovarian clear cell cancer cells ES-2.
Figure 13 shows the in vivo tumor inhibition effect of the B7H3/PDL1 bispecific antibody of the present application.
Figure 14 shows the effects of the B7H3/PDL1 bispecific antibody of the present application on the body weight of mice.

### Detailed embodiments

The present invention is further described below with reference to specific embodiments, but the present invention is not limited to the examples.

Unless otherwise specified, the materials, reagents, instruments and methods used in the following examples are conventional materials, reagents, instruments and methods in the art, and are commercially available.

### Examples

The present invention is described through specific embodiments so that those skilled in the art can better understand the technical contents of the present application, but these embodiments should not be understood as limiting the scope of the present invention.

### Preparation Example

### 1. Humanization of camel-derived anti-B7H3

The camel-derived anti-B7H3 nanobody was humanized by the framework shuffling method using the germline gene of the human antibody gene as a template. The corresponding VHH framework-shuffled libraries were all generated by full in vitro synthesis using overlap PCR. Then the clones of the VHH phage library were constructed, screened and identified.

In specific, the camel-derived anti-B7H3 nanobody was humanized using a one-step strategy. About 1,000 clones were screened from the sublibrary, and the selected positive clones were screened for phage-level thermostability using ELISA assay. A high adsorption 96-well ELISA plate was coated with 5 µg/ml huB7H3 antigen, and after the screened phage was amplified overnight, the supernatant was reacted to select clones with a higher OD450 reading.

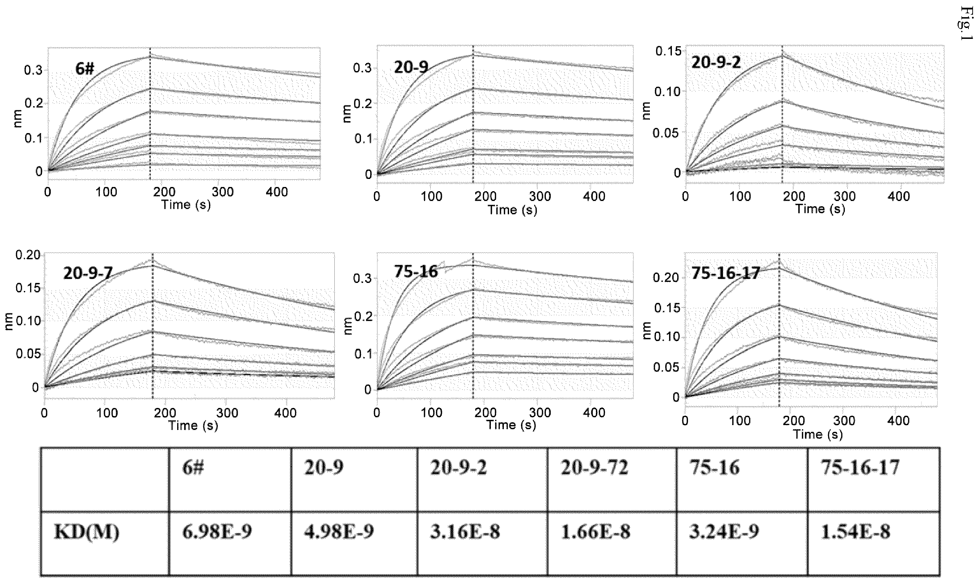

The above phage clones were sequenced to obtain the B7H3 VHH gene sequence, and its C-terminus was fused with the human Fc protein gene to construct and express B7H3-Fc. 100nM of B7H3 VHH-Fc was captured by the biolayer interferometry method using the Protein A probe and bound to B7H3 antigen with an initial concentration of 200nM, a 2-fold dilution. The KD value of the antibody binding to antigen was calculated. The results showed that 75-16 (whose amino acid sequence is set forth in SEQ ID NO.: 15 with CDR1 sequence of SEQ ID NO.: 12, CDR2 sequence of SEQ ID NO.: 13, and CDR3 sequence of SEQ ID NO.: 14) has the highest affinity with B7H3, with a KD of up to 3.24×10⁻⁹ M (see Figure 1), so it was selected for constructing the bispecific antibody in the next step.

### 2. Construction and expression of the B7H3/PDL1 bispecific antibody

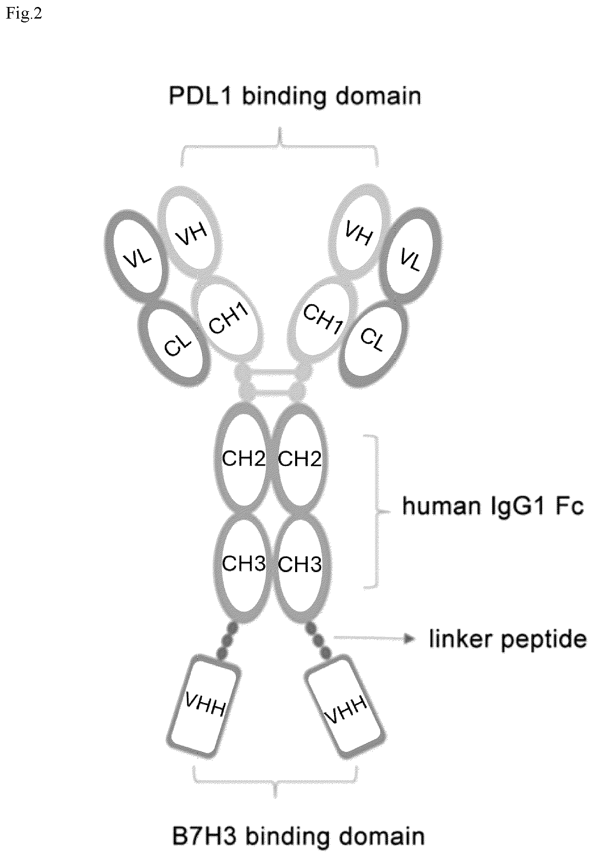

The amino acid sequences of the light chain (with an amino acid sequence of SEQ ID NO.: 9, wherein the amino acid sequence the variable region sequence is SEQ ID NO.: 4, the amino acid sequence of CDR1 is SEQ ID NO.: 1, the amino acid sequence of CDR2 is SEQ ID NO.: 2, and the amino acid sequence of CDR3 is SEQ ID NO.: 3) and heavy chain (with an amino acid sequence of SEQ ID NO.: 10, wherein the amino acid sequence the variable region sequence is SEQ ID NO.: 8, the amino acid sequence of CDR1 is SEQ ID NO.: 5, the amino acid sequence of CDR2 is SEQ ID NO.: 6, and the amino acid sequence of CDR3 is SEQ ID NO.: 7) of PDL1 monoclonal antibody were derived from the existing PDL1 hlgG1 humanized monoclonal antibody, and the C-terminus of the anti-B7H3 VHH (75-16 above) was linked to the C-terminus of the Fc fragment via a linker peptide (SEQ ID NO.: 11) (the structure is shown in Figure 2).

The DNA sequence was synthesized, subcloned into the pcDNA3.1 vector and amplified in Escherichia coli. The purified plasmid was transfected into HEK293 cells by PEI. The cells were then suspended in OPM-CD05 expression medium for culture. After 6 days of culture, the cell culture supernatant was collected and the antibody was purified by protein A column. The purified IgG1 was dialyzed with phosphate buffered saline (PBS), snap-frozen and stored at -80°C.

The amino acid sequence of the heavy chain of the purified B7H3/PDL1 bispecific antibody is set forth in SEQ ID NO.: 16, and the amino acid sequence of its light chain is set forth in SEQ ID NO.: 17.

### Tests

Hereinafter, unless otherwise specified, the term "bispecific antibody" refers to the B7H3/PDL1 bispecific antibody in the present application, which is also referred to as "B7H3/PDL1 bispecific antibody" or "bispecific antibody".

### Method:

### Example 1 Sodium dodecyl sulfate-polyacrylamide gel electrophoresis (SDS-PAGE)

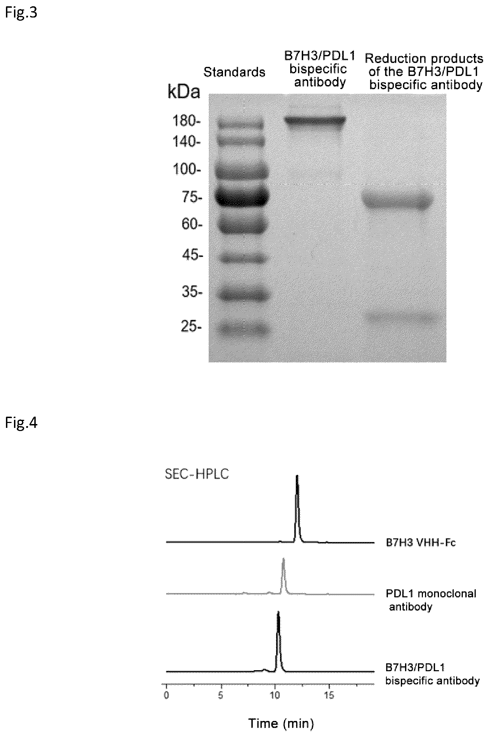

5µg bispecific antibody was mixed with protein reducing and non-reducing buffers and the system was supplemented with PBS to a volume of 10µl. After heating at 100 °C for 10 minutes to fully denature the protein, 9µl of the solution was added to the prefabricated polyacrylamide gel well (Bio-Rad). The separation was carried out at a voltage of 80V for 30 minutes and then 120V for 60 minutes, and then the gel was stained with Coomassie brilliant blue staining solution for 30 minutes, decolorized with a decolorizing solution (acetic acid: ethanol: water = 1:3:6) for 15 minutes for three times such that the background was faded, and then the gel was photographed with a gel imager. The results showed that the B7H3/PDL1 bispecific antibody had good monomer purity under non-reducing conditions. Under reducing conditions, two bands of heavy chain and light chain appeared due to the breakage of the disulfide bonds between the light and heavy chains, and there were no impurity band (Figure 3).

### Example 2 Size Exclusion Chromatography (SEC-HPLC)

SEC-HPLC analysis was used to evaluate the monomer purity of the bispecific antibody. The B7H3/PDL1 bispecific antibody was analyzed on a Thermo MAbPac SEC-1, 5 µm, (7.8 × 300 mm) P/N 088460 using a 1260 HPLC system (Agilent, Santa Clara, CA) and compared with PDL1 monoclonal antibody and B7H3 VHH-Fc. The mobile phase used was phosphate buffered saline (PBS). The flow rate was set to 0.7 mL/min; injection volume: 15 µl. As shown in the SEC chromatogram (Figure 4) which was recorded by monitoring the absorbance at 280 nm using a UV detector at a constant temperature of 25 °C, the detected antibodies all had a very high proportion of monomer peaks, and the peak area ratio of the monomer peak is more than 95%, which indicated that the bispecific antibody had good monomer purity and a small number of aggregates under PBS buffer conditions.

### Example 3 Determination of Tm value of the antibody by differential scanning fluorimetry (DSF)

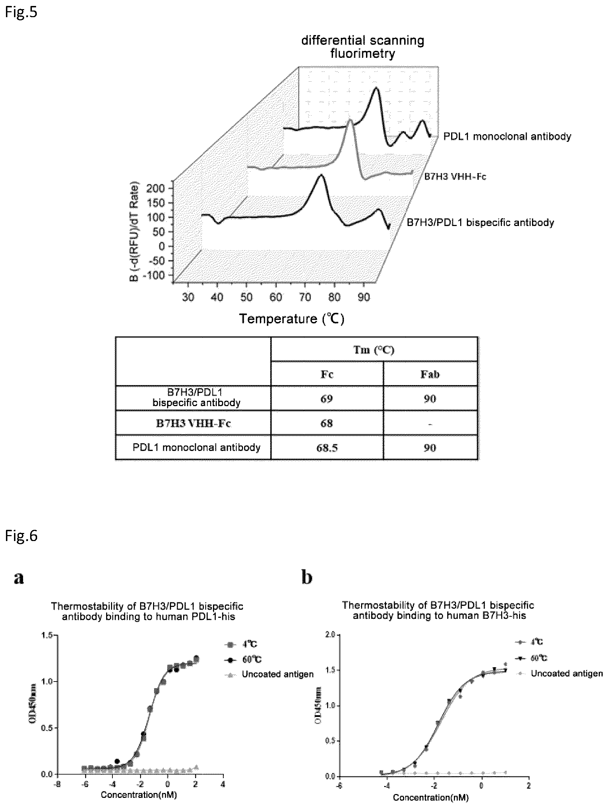

DSF was detected by a real-time PCR instrument (Biorad cfx96, USA). The B7H3/PDL1 bispecific antibody, B7H3 VHH-Fc, and PDL1 monoclonal antibody were diluted to 1 mg/mL in PBS. SYPRO Orange was diluted 1000-fold from a 5000-fold concentrated stock solution with ddH₂O. 20µl of the sample was added to a PCR tube, and added with a SYPRO Orange working solution to prevent bleaching. The tube was immediately centrifuged to pool the liquid at the bottom of PCR tube. The qPCR instrument was turned on, and heated programmably from 25 °C to 95 °C at a 0.3 °C/sec heating rate. The data were collected, and signal values were plotted against temperature, and melting temperature (Tm) was calculated. The data showed that the Tₘ of Fc and Fab of the B7H3/PDL1 bispecific antibody were 69 °C and 90 °C respectively, achieving good high temperature resistance, which were close to the Tm of B7H3 VHH-Fc and PDL1 monoclonal antibody (Figure 5).

### Example 4 Evaluation of the thermostability and binding activity of the antibody by binding ELISA

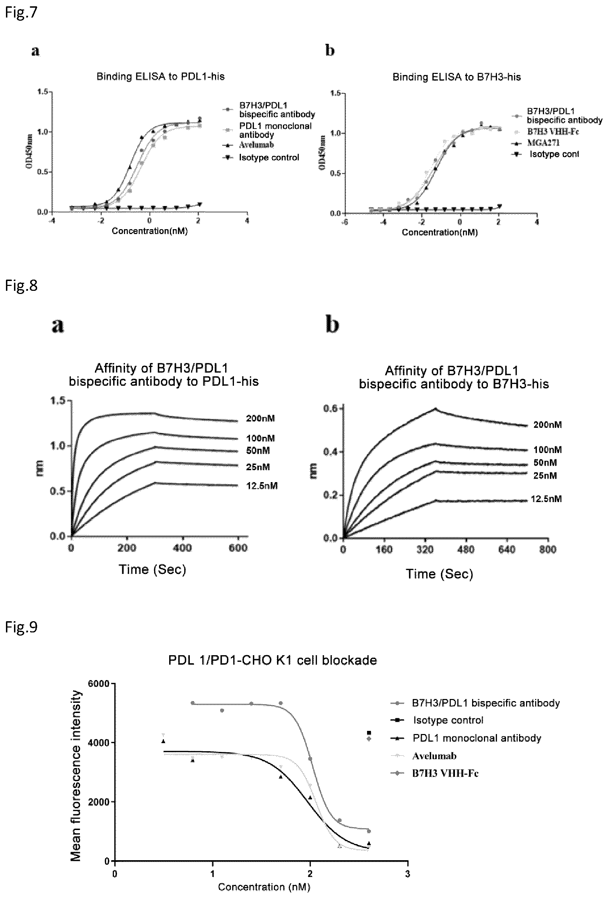

A 96-well ELISA plate was coated with 2 µg/ml of his-tagged PDL1 or B7H3 antigen protein at 4°C overnight, and 100 µl of Casein blocking solution was added to each well the next day for blocking at 37 °C for 1 hour. Three-fold dilutions of the B7H3/PDL1 bispecific antibody (when testing thermostability, the bispecific antibody was treated in a 60 °C water bath for 1 hour) and monoclonal antibodies (when testing the binding of bispecific antibody to B7H3 by ELISA, the control monoclonal antibodies used were MGA271, Isotype hlgG1, B7H3 VHH-Fc; when testing the binding to PDL1, the control monoclonal antibodies were PDL1 monoclonal antibody, Avelumab, and Isotype hlgG1) were added to the plate. After incubation at 37 °C for 1 hour, unbound antibodies were washed off with 0.1% PBST, and bound antibodies were detected using horseradish peroxidase (HRP)-conjugated goat anti-human IgG (H+L) antibody (Jackson ImmunoResearch, USA), and 50 µl of 3,3',5,5'-tetramethylbenzidine substrate (TMB) was used for color development. After standing for 5 minutes, 50 µl of 2 M sulfuric acid was added to stop the color development, and then the absorbance was detected using a SpectraMax M5e (Molecular Devices) microplate reader at OD450 nm. The antibody concentration was plotted against the OD450 reading using a 4-parameter fitting method, and the EC₅₀ was calculated. Figures 6a and 6b showed the binding curves of the bispecific antibody with PDL1 and B7H3 before and after treatment at 60°C for 1 hour. It can be seen from the figures that the binding curves of the bispecific antibody before and after heating basically overlapped, regardless of whether it is for PDL1 or B7H3, which indicated that the bispecific antibody could withstand the high temperature of 60°C and retain strong binding activity to the dual targets of PDL1 and B7H3. On the other hand, the bispecific antibody had similar binding ability to that of the positive antibody, PDL1 monoclonal antibody, and B7H3 VHH-Fc for both targets. The EC₅₀ of the bispecific antibody, PDL1 monoclonal antibody, and control Avelumab binding to human PDL1 were 0.3056 nM, 0.4407 nM, and 0.1563 nM, respectively, which were of the same order of magnitude (Figure 7a); the bispecific antibody, B7H3 VHH-Fc, and MGA271 also had similar binding activities to human B7H3, with EC₅₀s of 0.04105nM, 0.02515nM, and 0.05476nM, respectively (Figure 7b).

### Example 5 Determination of the affinity of the bispecific antibody binding to antigen by BLI

The B7H3/PDL1 bispecific antibody, PDL1 monoclonal antibody, and B7H3 VHH-Fc were diluted to 100 nM in a sample buffer (0.02% tween 20 and 0.1% BSA in PBS), and the affinity of the bispecific antibody to specific human B7H3 and human PDL1 antigens was analyzed by OCTET 96, wherein the probe used was Protein A material to fix the antibody; B7H3-his and PDL1-his antigens were diluted to an initial concentration of 200 nM with the sample buffer, and multiple antigen gradients were set at a 2-fold dilution for binding to antibodies and rate constants and affinity were obtained. Kon and Koff values were calculated using the software provided by the supplier to obtain the KD value of the antibody. It can be seen from the figure that the bispecific antibody had a high affinity for PDL1 and B7H3, with KD values reaching 5.85×10⁻¹⁰ M and 8.11×10⁻⁹ M, respectively (Figure 8).

### Example 6 Detection of the capacity of the bispecific antibody to block the PD1/PDL1 pathway by Flow cytometry

The capacity of the B7H3/PDL1 bispecific antibody to block the binding of human PDL1 to human PD1-CHO cells was evaluated by flow cytometry and compared with Avelumab and PDL1 monoclonal antibody. 2×10⁵ CHO-PD1 cells were evenly plated in a 96-well culture plate and incubated with a mixture of antibodies (B7H3/PDL1 bispecific antibody, Avelumab, PDL1 monoclonal antibody, Isotype hlgG1, B7H3 VHH-Fc), starting from 400nM, serially diluted and biotinylated PDL1 antigen (50nM) at room temperature for 30 minutes. The mixed solution was then incubated with the cells at 4 °C for 45 minutes, and unbound antigens were washed off with PBS.

The cells were then fluorescently stained with PE-streptavidin. Finally, the mean fluorescence intensity (MFI) of the PE channel in the flow cytometer was read, the antibody concentration was plotted against MFI, and the IC₅₀ of the antibody was calculated using a four-parameter fitting. The results of flow cytometry analysis showed that the bispecific antibody was able to block the binding of PDL1 to CHO-PD1 cells, with an IC50 of 106.4 nM and a blocking activity similar to that of PDL1 monoclonal antibody (IC₅₀ of 94.20 nM) and Avelumab (IC₅₀ of 115.0 nM) (Figure 9).

### Example 7 Determination of the ability of bispecific antibody to activate T cells by Mixed lymphocyte reaction (MLR)

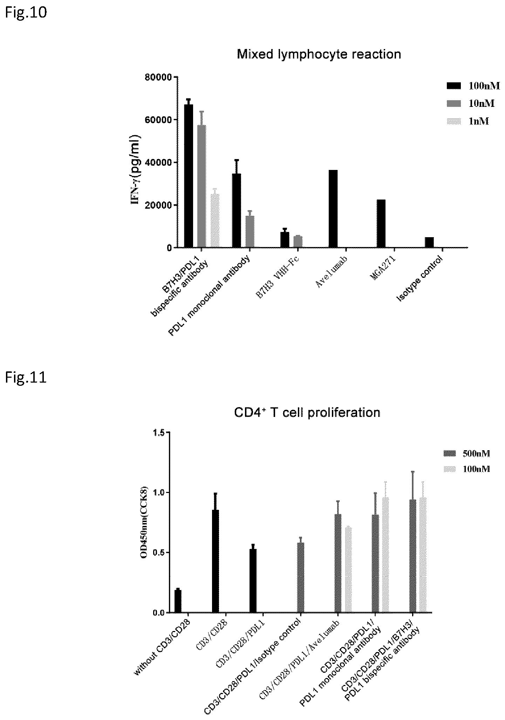

Monocytes were isolated from peripheral blood mononuclear cells (PBMCs) which were cultured in vitro for 7 days using a monocyte purification kit (Miltenyi Biotec, Germany) with 500 U/mL interleukin-4 (IL-4) and 250 U/mL GM-CSF to induce the production of dendritic cells (DCs). CD4⁺ T cells (1×10⁵) and allogeneic DCs (1.25×10⁴) were co-cultured in a RPMI 1640 complete medium containing 10% FBS, with 5% CO₂ at a constant temperature of 37°C and groups without antibody and with different concentrations of B7H3/PDL1 bispecific antibody, PDL1 monoclonal antibody, Avelumab, B7H3 VHH-Fc, MGA271, and Isotype hlgG1 were set up. After 5 days, the IFN-γ concentration in the culture supernatant was analyzed using an IFN-y ELISA detection kit. MLR results showed that the bispecific antibody could stimulate CD4⁺T cells to secrete IFN-y, and the T cell activation ability of the bispecific antibody was superior to that of PDL1 monoclonal antibody and Avelumab at low or high concentrations; in addition, we further observed that in the presence of B7H3 antibody alone, although less pronounced compared to PD-L1 blockade, partial T cell activation was achieved through inhibition of the B7H3-mediated inhibitory signaling pathway (Figure 10)).

### Example 8 T cell proliferation assay

1µg/ml of CD3 antibody (Clone HIT3a), 1µg/ml of CD28 antibody (Clone CD28.2) and 5µg/ml of human PDL1 were coated on 96-well cell plates (Corning, USA) at 4 °C for 1 hour, and control wells were coated with mouse IgG2a isotype control alone or with CD3 and CD28 antibodies using the same method. CD4⁺ T cells were isolated using Dynabeads^{™} CD4 Positive Isolation Kit. CD4⁺ T cells were cultured with different concentrations of B7H3/PDL1 bispecific antibody, Avelumab and PDL1 monoclonal antibody in RPMI1640 medium containing 10% FBS (Gibco) in pre-coated 96-well plates at 37 °C for 4 days. After 4 days, the CCK8 kit was used to detect the changes of the number of T cells. The data showed that freshly isolated human CD4⁺ T cells which were cultured on well plates coated with anti-CD3 and anti-CD28 antibodies showed increased proliferation. When PDL1 was added to the wells, the proliferation ability was significantly reduced, which confirms that PDL1 provided inhibitory signals to T cells; Avelumab, PDL1 monoclonal antibody and the bispecific antibody could significantly promote T cell proliferation at concentrations of 100nM and 500nM (Figure 11).

### Example 9 Antibody-dependent cell cytotoxicity (ADCC)

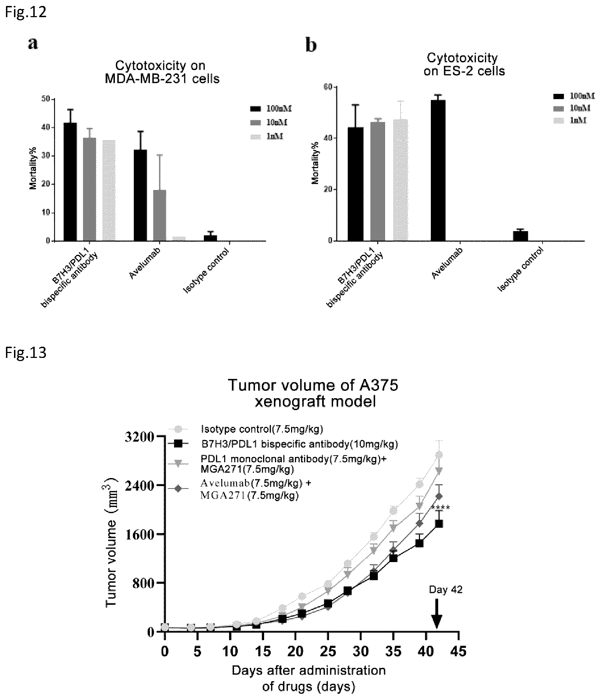

The main anti-tumor effects of MGA271 and Avelumab are due to the ADCC effects of the antibodies, which is related to its IgG1 subtype. The bispecific antibody also has an IgG1 functional region. The ADCC of the antibody was measured using the LDH cytotoxicity detection kit, and human PBMCs were purified from the leukocytes using Ficoll gradient centrifugation and NK cells were isolated from human PBMCs using negative selection magnetic beads (Miltenyi Biotec, Auburn, CA). NK cells (3×10⁶) and MDA-MB-231 and ES-2 cells (3×10⁵) were co-cultured with or without different concentrations of bispecific antibodies and Avelumab at the beginning of the assay. After 18 hours, lactate dehydrogenase (LDH) secretion in the culture supernatant was analyzed by ELISA. When B7H3⁺ PDL1⁺ MDA-MB-231 was used as the target cell, both the bispecific antibody and Avelumab showed ADCC activity, but the activity of the bispecific antibody was stronger at low concentrations; when using PDL1⁺ ES-2 cells as the target cell, it was observed that the activity of the bispecific antibody was comparable to that of Avelumab at a dose of 100 nM, and it also showed strong ADCC activity at low concentrations (Figure 12).

### Example 10 Study on the in vivo antitumor activity of the bispecific antibody

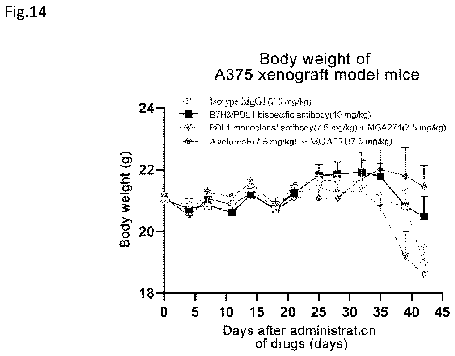

Human PBMC (6.67×10⁶) was injected into 41 NPSG mice through the tail vein one day before inoculation of A375 tumor cells, and 5×10⁶ A375 tumor cells were injected subcutaneously the next day. Five days later, subcutaneous tumors were observed. The mice were divided into groups of isotype control group, monoclonal antibody combination group (Pembrolizumab+MGA271 and Avelumab+MGA271), monoclonal group and bispecific antibody group, with 10 animals/group. After the model was successfully established, the drug was administered on the 5th day, twice a week until the end of the experiment. Tumor volume and animal body weight were measured and recorded on days 0, 4, 7, 11, 14, 18, 21, 25, 28, 32, 35, 39 and 42 of the experiment. The efficacy and safety were evaluated by the tumor growth inhibition value based on the relative tumor volume (TGIRTV) and the change in animal body weight. The bispecific antibody maintained significantly stronger activity than the PD1 monoclonal antibody + MGA271 combination group from beginning to end; the bispecific antibody gradually showed an effect superior to the Avelumab + MGA271 group over time, and the TGls at the end of the experiment were 39.29% and 26.45% respectively (Figure 13); in addition, there was no serious body weight loss of mice during the experiment, proving the safety of the bispecific antibody in the treatment (Figure 14).

From the test results of the above test examples, it can be seen that the bispecific antibody constructed in the present application can bind to B7H3 and PDL1 at the same time, and can relieve the inhibition of PDL1 on T cells while targeting tumor cells, and at the same time shows anti-tumor activity superior to the combination of monoclonal antibodies.

## Claims

1. A B7H3/PDL1 bispecific antibody targeting B7H3 and PDL1, and the bispecific antibody comprises:
a monoclonal antibody unit, which targets PDL1 and comprises 2 heavy chains and 2 light chains;
a nanobody unit, which targets B7H3 and comprises 2 identical nanobodies,
wherein the C-termini of the two nanobodies are linked to the C-termini of the Fc fragments of the two heavy chains of the monoclonal antibody unit via a linker peptide, respectively.

2. The bispecific antibody of claim 1, wherein the linker peptide is a polypeptide comprising a glycine and a serine and having certain elasticity and protease resistance, preferably, the amino acid sequence of the linker peptide is set forth in SEQ ID No.: 11.

3. The bispecific antibody of claim 1, wherein
the light chain variable region of the monoclonal antibody unit comprises CDR1 with an amino acid sequence of SEQ ID NO.: 1, CDR2 with an amino acid sequence of SEQ ID NO.: 2, and CDR3 with an amino acid sequence of SEQ ID NO.: 3, the heavy chain variable region of the monoclonal antibody unit comprises CDR1 with an amino acid sequence of SEQ ID NO.: 5, CDR2 with an amino acid sequence of SEQ ID NO.: 6, and CDR3 with an amino acid sequence of SEQ ID NO.: 7, and the nanobody comprises CDR1 with an amino acid sequence of SEQ ID NO.: 12, CDR2 with an amino acid sequence of SEQ ID NO.: 13, and CDR3 with an amino acid sequence of SEQ ID NO.: 14;
preferably, the light chain variable region of the monoclonal antibody unit comprises an amino acid sequence set forth in SEQ ID NO.: 4, and the heavy chain variable region of the monoclonal antibody unit comprises an amino acid sequence set forth in SEQ ID NO.: 8; and the nanobody comprises an amino acid sequence set forth in SEQ ID NO.: 15;
Further preferably, the light chain of the monoclonal antibody unit comprises an amino acid sequence set forth in SEQ ID NO.: 9, and the heavy chain of the monoclonal antibody unit comprises an amino acid sequence set forth in SEQ ID NO.: 10, and the nanobody comprises an amino acid sequence set forth in SEQ ID NO.: 15;
More preferably, the full-length amino acid sequence of the light chain of the monoclonal antibody unit is set forth in SEQ ID NO.: 9, and the full-length amino acid sequence of the heavy chain of the monoclonal antibody unit is set forth in SEQ ID NO.: 10, and the amino acid sequence of the nanobody is set forth in SEQ ID NO.: 15.

4. The bispecific antibody of claim 1, wherein the amino acid sequence of the heavy chain of the bispecific antibody is set forth in SEQ ID NO.: 16, and the amino acid sequence of the light chain of the bispecific antibody is set forth in SEQ ID NO.: 17.

5. A polynucleotide encoding the B7H3/PDL1 bispecific antibody of any one of claims 1 to 4.

6. An expression vector comprising the polynucleotide of claim 5.

7. A pharmaceutical composition comprising a therapeutically effective amount of the B7H3/PDL1 bispecific antibody of any one of claims 1 to 4, and a pharmaceutically acceptable carrier.

8. Use of the B7H3/PDL1 bispecific antibody of any one of claims 1 to 4 in the preparation of a drug for the prevention, diagnosis, treatment or adjuvant treatment of tumors.

9. The use of claim 8, wherein the drug inhibits tumors by binding to B7H3, blocking the B7H3 signaling pathway, and thereby mediating the ADCC effect, or
the drug inhibits tumors by binding to PD-L1, blocking the binding of PD-1 to PDL-1, activating T lymphocytes, and increasing the expression of IL-2, IFN-y in T lymphocytes,
preferably, the drug inhibits tumors by binding to B7H3, blocking the B7H3 signaling pathway, and by binding to PD-L1, blocking the binding of PD-1 to PDL-1, activating T lymphocytes, and increasing the expression of IL-2, IFN-y in T lymphocytes.

10. The use of claim 8, wherein the tumor is selected from one or more of lung cancer, gastric cancer, liver cancer, colorectal cancer, melanoma, kidney tumor, ovarian cancer, prostate cancer, bladder cancer, breast cancer, esophageal cancer, colorectal cancer, nasopharyngeal cancer, brain tumor, cervical cancer, blood cancer, bone cancer, lymphoma, pancreatic cancer and Ewing's sarcoma, preferably, the tumor is breast cancer, ovarian cancer or melanoma.
